# EUROPEAN PATENT SPECIFICATION

(11) **EP 2 376 239 B1**
(45) Date of publication and mention of the grant of the patent: **20.02.2013**
(21) Application number: 09798959.4
(22) Date of filing: 01.12.2009
(51) Int. Cl.: B06B 1/02

(54) **ULTRASOUND TRANSDUCER PROBE WITH FRONT-END CIRCUIT**
ULTRASCHALLWANDLERSONDE MIT EINGANGSSCHALTUNG
SONDE À TRANSDUCTEUR ULTRASONIQUE POURVU D'UN CIRCUIT D'ENTRÉE

(30) Priority: 10.12.2008 US 121284 P
(43) Date of publication of application: 19.10.2011
(73) Proprietor: Koninklijke Philips Electronics N.V., 5621 BA Eindhoven (NL)
(72) Inventor: OLSSON, Lars J., NL-5656 AE Eindhoven (NL); ROBINSON, Andrew, NL-5656 AE Eindhoven (NL); BETTS, Richard, NL-5656 AE Eindhoven (NL)
(74) Representative: Van Velzen, Maaike Mathilde
(86) International application number: PCT/IB2009/055433
(87) International publication number: WO 2010/067258

(56) References cited:
- WO-A2-2007/017775
- DE-A1- 10 336 101
- US-A- 4 725 993

## Description

### FIELD OF THE INVENTION

The present invention relates to an ultrasound transducer probe (scanhead) having an array of transducer elements for transmitting ultrasound transmit pulses and receiving echo signals in response to these transmit pulses. More precisely, the invention refers to a front-end circuit preconnected to such an ultrasound transducer probe, wherein said front-end circuit, which may e.g. be realized as an application-specific integrated circuit (ASIC) with given input voltage constraints prescribing a limited supply voltage, comprises a transmission stage, said transmission stage comprising a branched voltage control line or lines with two transmit branches being respectively connected to a different terminal of each transducer element for providing each of these transducer elements with a differential excitation or pulse voltage whose amplitude level is up to twice the voltage level of the single-ended front-end circuit which is supplied by said voltage control line. In this context, a bridged amplifier topology is proposed which comprises at least one transmit amplifier or pulser integrated in each one of the two transmit branches, wherein the transmit amplifier in a first one of these transmit branches provides an output signal corresponding to the non-inverted input signal and the transmit amplifier in a second one of said transmit branches provides an output signal corresponding to an inverted form of said input signal such that up to twice the voltage amplitude of the ultrasound transducer's front-end supply voltage is lying across each transducer element without needing to provide this doubled voltage level at the voltage supply inputs of the application-specific integrated circuit, thus being able to use the same IC fabrication process to get twice the voltage swing over the transducer elements.

### BACKGROUND OF THE INVENTION

Ultrasound medical diagnostic systems are used to generate sonography images of anatomical structures within a patient's body by scanning a target area with ultrasound signals. Typically, ultrasound signals in the order between 2.0 MHz and 10 MHz are transmitted into a patient via an ultrasound transducer probe. The transmitted ultrasound energy is in part absorbed, dispersed, refracted, and reflected by the patient's body, and the reflected ultrasound energy is received at the transducer probe where it is converted into electronic echo signals which can be evaluated and further processed. In many conventional ultrasound systems, it may e.g. be provided that received echo signals undergo a beamforming. Subsequently, the beamformed signals may be processed to analyze echo, Doppler, and flow information and to obtain a sonography image of a targeted anatomy structure or tissue region of interest in the interior of the patient's body.

WO 2007/017775 describes an ultrasonic transducer, a transducer array and an ultrasonic probe.

DE 103 36 101 A describes a system including a transistor connected with a transducer element and an amplifier input, and another transistor connected with the transducer element and a power source. The transistors are connected with same electrode of the transducer element.

### SUMMARY OF THE INVENTION

In conventional designs of compact portable ultrasound machines which are available on the market today, the front-end circuit may be equipped with a transmitter unit implemented by an integrated circuit with given input voltage constraints prescribing a limited supply voltage, and it may be that the process used to fabricate this integrated circuit is not able to handle those high voltage levels which are usually required and desired for traditional ultrasound probes to get sufficient acoustic transmit power. For other applications, e.g. catheters and internal probes, such as e.g. endoscopic ultrasound probes for use in transesophageal echocardiography (TEE), it might be necessary to use probes with lower voltages in order to reduce any potential risks to the patient and to minimize the probe's size by providing thinner insulation layers.

An object of the present invention is thus to find a way to integrate the transmitting part (and also the receiving part) of a front-end circuit into an ultrasound machine while considering the above-mentioned constraints concerning the front end circuit's output power and the integrated circuit's input voltage. In this context, the present invention particularly aims at solving the problem of obtaining a higher transmit voltage without needing to change the process used to fabricate the front-end's integrated circuits.

In view of this object, a first exemplary embodiment of the present application refers to a front-end circuit of an ultrasound transducer probe having an array of differentially connected transducer elements for transmitting ultrasound transmit pulses and receiving echo signals in response to these transmit pulses, wherein said front-end circuit comprises a transmission stage with two separate transmit branches being respectively connected to a different terminal of each transducer element for providing each of these transducer elements with a differential excitation or pulse voltage whose amplitude level is given by the difference of the front-end circuit's input control signals which are fed via the two transmit branches to the respective transducer element.

According to a first aspect of this embodiment, the proposed front-end circuit may further comprise a bridged amplifier topology having at least one transmit amplifier integrated in each one of the two transmit branches which are used for providing each transducer element with the differential excitation or pulse voltage, wherein the transmit amplifier in a first one of these transmit branches provides a first output signal which is given by one of the front-end circuit's input control signals in a non-inverted form after being amplified by a gain factor and wherein the transmit amplifier in a second one of these transmit branches provides a second output signal which is given by the same input control signal in an inverted form after being amplified by the same gain factor. For example, if said input control signal is given by an input voltage, it can thus be provided that the amplitude level of the differential excitation or pulse voltage is up to twice the voltage level of this input voltage.

The transmit amplifiers may thereby be implemented as two linear amplifiers that are controlled by a linear input control signal. In this case (which represent one preferred embodiment of the present invention), the non-inverted input control signal is supplied to one amplifier and the inverted input control signal is fed to the other amplifier. The same effect could be achieved by having one of the two amplifiers be an inverting amplifier, in which case both amplifiers could be supplied with the same input control signal.

Alternatively, according to a second aspect of this embodiment, the proposed front-end circuit may comprise a bridged amplifier topology having at least one transmit pulser integrated in each one of the two transmit branches which are used for providing each transducer element with the differential excitation or pulse voltage, wherein the transmit pulser in a first one of these transmit branches provides a first output signal whose amplitude level is set by a first set of digital control signals fed to an input terminal of the transmit pulser in this first transmit branch and wherein the transmit pulser in a second one of these transmit branches provides a second output signal whose amplitude level is set by a second set of digital control signals fed to an input terminal of the transmit pulser in this second transmit branch.

The particular amplifiers (or pulsers) are thereby operated in a bridged mode. Each transducer element is thus connected across the output ports of two associated amplifiers and is supplied with up to twice the voltage level of the input voltage fed to said amplifiers' voltage supply terminals. Using such a circuit topology means in effect to double the number of used amplifiers. The proposed circuit makes it possible to double the amplitude level of the single-ended supply voltage at the supply port of the front-end circuit with the same IC process and further implies the advantage of obtaining a higher transmit power and therefore increased penetration or signal-to-noise ratio without needing to change the existing scanhead acoustic design and without needing to increase the amplitude level of the front-end circuit's supply voltage relative to a ground potential. Furthermore, the proposed front-end circuit allows the use of a less expensive scanhead design in case it is intended to maintain transmit power instead of increasing it or to do trade-offs between transmit power, penetration, signal-to-noise ratio and acoustic stack design and cost.

According to a specific aspect of the present invention, it may further be provided that the output ports of the transmit amplifiers or transmit pulsers are connected by a flip chip, flex circuit or other type of interconnect to an associated transducer element of the transducer array. That is, the front-end amplifiers are in the same package as the transducer elements in the scanhead, thus making a long cable connection between the amplifiers and the scanhead unnecessary.

Preferably, according to a specific aspect of the present invention, it may be foreseen that the transmit amplifiers or transmit pulsers are integrated in the ultrasound transducer probe and that the proposed front-end circuit is implemented as an application-specific integrated circuit of the ultrasound transducer probe.

According to the present invention, the proposed front-end circuit may further comprise a differential reception stage which provides an output signal representing said echo signals. The reception stage may thereby connect each terminal of the at least one transducer element to an associated low-noise amplifier.

Aside therefrom, the present invention also refers to a new type of ultrasound transducer probes. Whereas conventional scanheads are equipped with single-ended transducer elements having a common ground potential, said transducer elements being supplied with two wires for ground and signal, it may be provided that the proposed ultrasound transducer probe is equipped with differentially connected transducer elements, i.e. with transducer elements which do not have any ground electrode.

Therefore, a second exemplary embodiment of the present application relates to an ultrasound diagnostic imaging system which comprises an ultrasound transducer probe having an array of differentially connected transducer elements for transmitting ultrasound transmit pulses and receiving echo signals in response to these transmit pulses. According to the present invention, it may thereby be provided that said system is equipped with an integrated front-end circuit as described above with reference to said first exemplary embodiment.

According to a preferred aspect of this second exemplary embodiment, the claimed ultrasound diagnostic imaging system may advantageously be equipped with an integrated microbeamformer system.

### BRIEF DESCRIPTION OF THE DRAWINGS

These and other advantageous features and aspects of the invention will be elucidated by way of example with respect to the embodiments described hereinafter and with respect to the accompanying drawings. Therein,
- Fig. 1: shows the large-scale components of a conventional ultrasound imaging system according to the prior art,
- Fig. 2a: shows a conventional implementation of multiline beamforming in an ultrasound imaging system according to the prior art,
- Fig. 2b: shows a conventional implementation of sub-array multiline beamforming in another ultrasound imaging system according to the prior art, and
- Fig. 3a: shows an ultrasound transducer probe's front-end circuit according to the present invention,
- Fig. 3b: shows an analog implementation of the ultrasound transducer probe's front-end circuit depicted in Fig. 3a,
- Fig. 3c: shows a digital implementation of the ultrasound transducer probe's front-end circuit depicted in Fig. 3a,
- Fig. 4a: shows the waveform of a first analog voltage at the output terminal of a transmit amplifier stage in a first transmit branch of the ultrasound transducer probe's front-end circuit according to the analog implementation as depicted in Fig. 3b,
- Fig. 4b: shows the waveform of a second analog voltage at the output terminal of a transmit amplifier stage in a second transmit branch of the ultrasound transducer probe's front-end circuit according to the analog implementation as depicted in Fig. 3b, and
- Fig. 4c: shows the waveform of an excitation or pulse voltage for operating at least one differentially connected transducer element of the ultrasound transducer probe's transducer array, said excitation or pulse voltage being given by the difference of said first and second analog voltages.

### DETAILED DESCRIPTION OF THE PRESENT INVENTION

In the following, different embodiments of the present invention will be explained in detail with respect to special refinements and referring to the accompanying drawings.

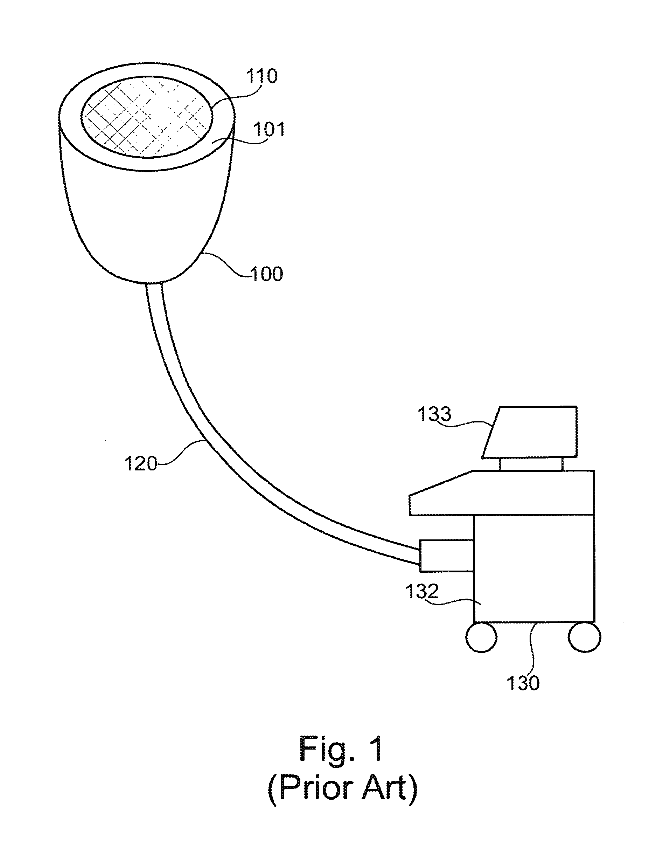

Fig. 1 depicts a conventional ultrasound system as known from WO 2006/035384 A1 that includes an ultrasonic transducer assembly (referred to in the art as "ultrasound transducer", "transducer probe" or "scanhead") 100. During an ultrasound examination, when generating sonography images, the attending physician holds an ultrasound transducer (a device that converts electrical signals into ultrasound signals to be transmitted and simultaneously receives reflected ultrasound waves) in his/her hand, places the transducer face (the scanhead or surface that emits sound waves and receives reflected sound waves) against a patient's skin and moved it over different portions of the patient's anatomy so as to obtain a set of desired sonography images. In order to ensure good contact between the transducer (sometimes also referred to as "probe" or "transducer probe") and the patient, a high viscosity water-soluble gel may be used such that the transducer glides over the patient's skin while the scanhead is emitting sound waves and picking up the back-scattered echoes. A computer will then analyze the echoes and display a sonography image on a monitor screen, and the shape and intensity of the echoes will depend on the density of the breast tissue. In ultrasound breast imaging, for example, if a fluid-filled cyst is being imaged, most of the sound waves will pass through the cyst and emit faint echoes. However, if a solid tumor is being imaged, the sound waves will bounce off the tumor, and the pattern of echoes will be translated by the computer into an image which will be recognized by the attending physician as indicating a solid mass. The patient may feel a slight pressure from the transducer, but she will not hear the highfrequency sounds.

Conventionally, ultrasonic transducers assemblies such as transducer probe 100 are connected to the base ultrasound system 130 by a cable 120. The base ultrasound system 130 comprises processing and control equipment 132, as well as the display 133. Those skilled in the art will note that the transducer probe could be readily constructed to include a wireless connection to the base ultrasound system in lieu of cable 120, and the software which drives the beamformer easily modified to receive and process the wireless signals from the transducer probe (e.g., radio transmission; see US 6,142,946).

System components that transmit and receive the ultrasonic waves in the transducer probe may be implemented differently in various ultrasonic systems. In the ultrasound system of Fig. 1, the face 101 of the transducer probe 100 (which is placed against the flesh of the subject to perform the imaging) includes an array 110 of piezoelectric elements (sometimes referred to as "transducer elements"), which both transmit and receive the ultrasonic waves. In ultrasound systems that use such arrays, the ultrasonic waves are created (and the resulting signals are interpreted) by a process called "beamforming", which process is performed mostly in signal processing hardware and software. When transmitting, individual piezoelectric elements in the transducer array 110 are stimulated in particular patterns in order to form and focus one or more ultrasonic beams. When receiving, the signal information received by individual piezoelectric elements in the transducer array 110 is delayed, combined, and otherwise manipulated in order to form electronic representations of one or more ultrasonic beams (i.e., beamforming).

One particular known beamforming practice is referred to as multi-line beamforming. In a "multiline beamforming", the transducer array 110 transmits a single ultrasonic beam, but the receive beamformer electronics synthesize several receive ultrasonic beams with different orientations. The oldest and most basic approach to multiline beamforming is to use multiple single line beamformers that are operated in parallel, such as described in US 4,644,795 to Augustine. In such an arrangement, each element in the transducer array is connected to a channel of the beamformer. Each of these channels applies delays to the signals from its corresponding element, which delays are appropriate to steer and focus the beam being formed by the beamformer. The signals delayed by each channel of the beamformer are combined to form a uniquely steered and focused beam, and the multiple beams which are produced simultaneously by parallel operated beamformers are used to form multiple lines of an ultrasound image.

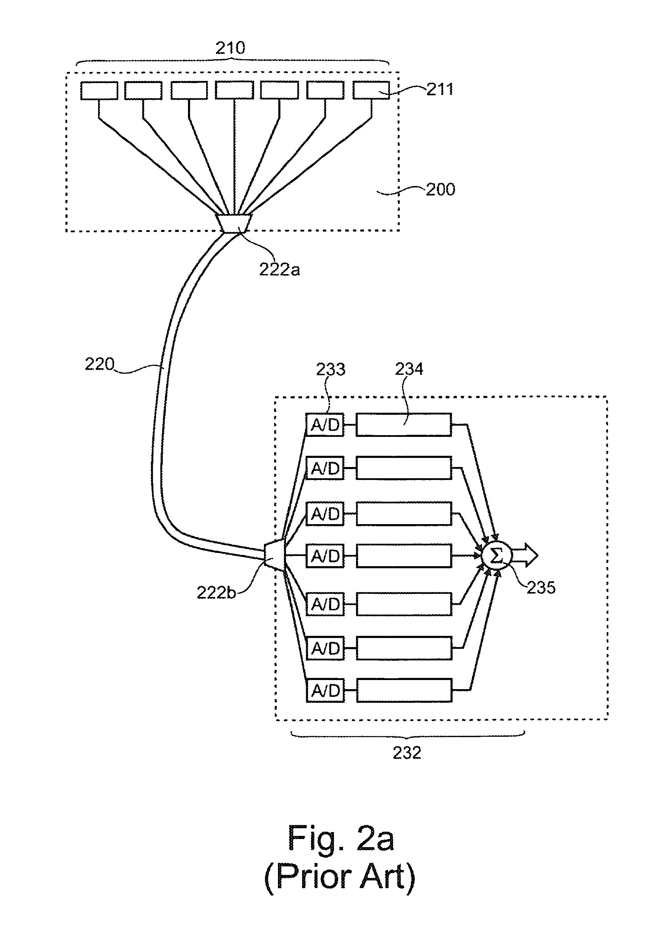

An example of an ultrasound imaging system with a conventional multiple signal line beamforming architecture as known from WO 2006/035384 A1 is shown in Fig. 2a, in which each of the transducer elements 211 of transducer array 210 (comprising ultrasound transducer probe 200) has a channel on which any received signals are transmitted over cable 220 to the processing means 232 in base ultrasound system 130. The signals received by the elements 211 may, or may not, be conditioned in the transducer (e.g., impedance matching) and then transmitted over cable 220 to the base ultrasound system. The processing means 232 takes the received signals, which are still in analog form, and converts them to digital signals using analog-to-digital (A/D) converters 233. The resulting digital signals are then delayed by digital delays 234 and summed together by summer 235 to form an acoustic receive sensitivity profile focused at any desired point within an imaging plane.

This approach is sufficient if the number of elements 211 being sampled in the transducer array 210 remains fairly low, i.e., under 200 or so elements (traditional beamformers have 128 channels). If the transducer array 210 has thousands of acoustic elements 211, the particular processing scheme requires that due to the use of samples from each of those elements, cable 220 would have to carry thousands of channels. Such a scheme would require a prohibitively large cable and more power than is available from a standard electric outlet (the typical power source for most ultrasound systems). For these and other reasons (including the excessive cost of such a cable and the associated electronics), the approach shown in Fig. 2a is not feasible when fully sampling the about 3000 elements which may be available in a transducer array.

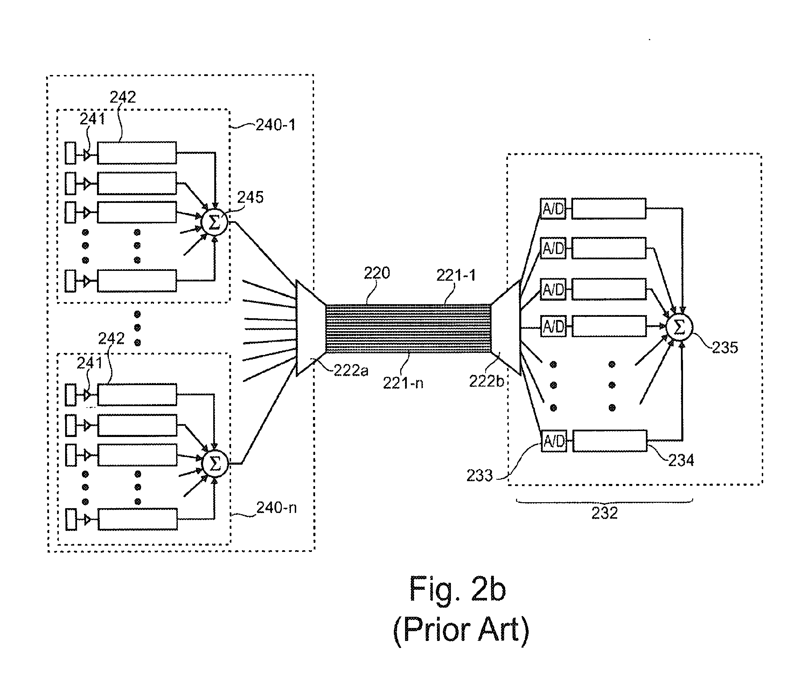

A known solution to this problem of complexity is referred to as "sub-array beamforming" or "micro-beamforming". An example of an ultrasound imaging system with a microbeamforming architecture as known from WO 2006/035384 A1, which is capable of implementing a microbeamforming process, is sketched in Fig. 2b. The detailed process is fully described in both the paper entitled "Fully Sampled Matrix Transducer for Real Time 3D Ultrasonic Imaging" by Bernard Savord and Rod Solomon (Paper 3J-1, Proceedings of the 2003 IEEE Ultrasonics Symposium, Oct. 5-8, 2003 (IEEE Press)), and in US 5,318,033. As described in the paper and US patent, and as shown in Fig. 2b, sub-array beamforming requires that the beamforming function be split into two stages, the first stage taking place in the transducer 200, and the second stage taking place in the processing means 232 of the base ultrasound system 130. By performing partial beamforming in the first stage inside transducer 200, the number of channels required to be transmitted over cable 220 to base ultrasound system 130 is drastically reduced.

As shown in Fig. 2b, individual elements 211 in transducer array 210 are grouped into sub-arrays 240-1 to 240-n. Each element 211 in each sub-array 240 has a pre-amplifier 241, and a low-power analog delay 242. Each sub-array 240 has a sub-array summer 245 for combining the appropriately delayed analog signals within the sub-array into one channel. Examples of low power analog delay technology which can be used in the first stage include mixers, phase shifters, charge coupled devices (CCD), analog random access memory (ARAM), sample-and-hold amplifiers, and analog filters, etc. All these technologies have sufficient dynamic range and use sufficiently low power to allow their integration into application-specific integrated circuits (ASICs), which ASICs are capable of fitting inside transducer 200 to carry out the microbeamforming application.

When performing microbeamforming, different bulk delays may be applied to each sub-array signal, where each bulk delay imposes the appropriate delay on each sub-array relative to the other sub-arrays. The partially beamformed analog signals from sub-arrays 240-1 to 240-n are transmitted on channels 222-1 to 222-n over cable 220 to processing means 232 in the base ultrasound system 130. The sub-array analog signals are converted to digital by A/Ds 233, appropriately delayed by digital delays 234, and then combined by final summer 235. The bulk delays discussed in the paragraph above may be implemented by digital delays 234.

Although contiguous, the transducer elements, which comprise a sub-array, may form a variety of shapes or patterns on the transducer array. For example, in a rectangularly shaped transducer array, each column of transducer elements may form a sub-array. Such constructions are described in US 6,102,863, US 5,997,479, US 6,013,032, US 6,380,766 and US 6,491,634. In the US 6,102,863 patent, "elevation" beamforming (i.e., combining the signals in each column of elements) is performed in the transducer, while "azimuth" beamforming (i.e., combining the row of previously combined columns) is performed by the processing means in the ultrasound system.

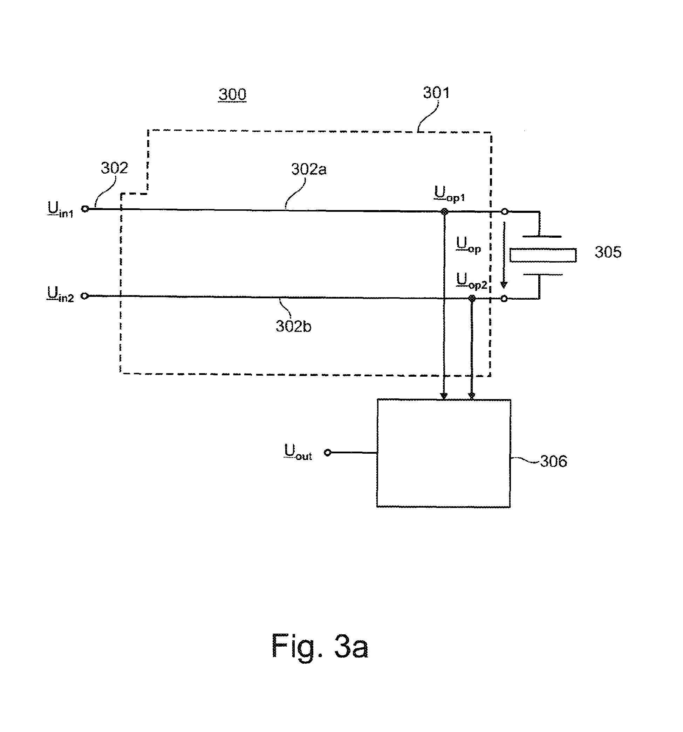

In Fig. 3a, an ultrasound transducer probe's front-end circuit 300 according to the present invention is shown, wherein said front-end circuit comprises a transmission stage 301 with two separate transmit branches 302a and 302b being respectively connected to a different terminal of each transducer element 110 (here given by a piezoelectric element 305) for providing each of these transducer elements with a differential excitation or pulse voltage *Uₒₚ* whose amplitude level is given by the difference of the front-end circuit's input voltages *U*_{*in*1} and *U*_{*in*2} which are fed via the two transmit branches 302a and 302b to the respective transducer element 110.

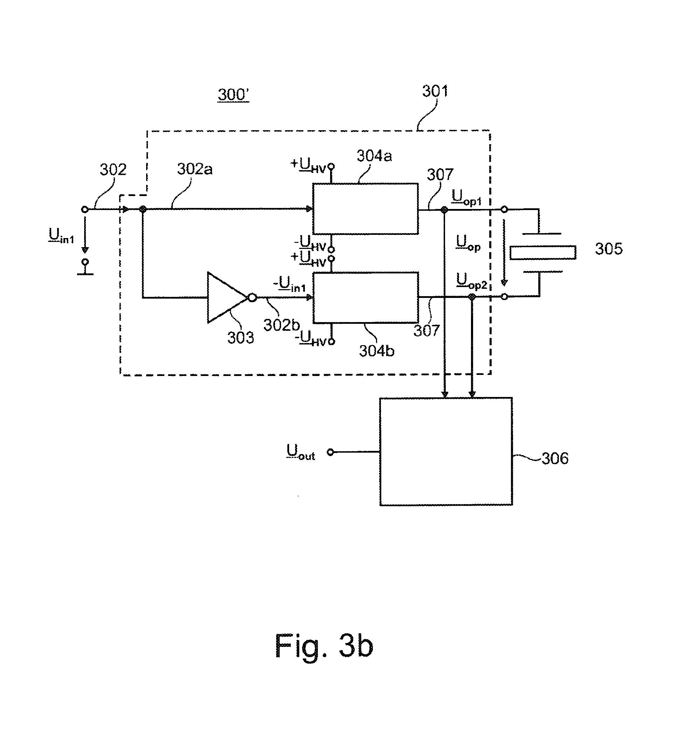

An analog implementation 300' of the ultrasound transducer probe's front-end circuit 300 as depicted in Fig. 3a is shown in Fig. 3b. Therein, a bridged amplifier topology is applied which comprises at least one transmit amplifier 304a, 304b integrated in each one of the two transmit branches 302a and 302b which are used for providing each transducer element 110 with the differential excitation or pulse voltage U p, wherein the transmit amplifier 304a in a first one (302a) of these transmit branches provides a first output signal *Uₒₚ*1 which is given by the front-end circuit's input voltage *U*_{*in*1} in a non-inverted form (*U*_{*op*1} *=* +*k·U*_{*in*1}) where k denotes the gain of amplifiers 304a and 304b and wherein the transmit amplifier in a second one (302b) of these transmit branches provides a second output signal *U*_{*op*2} which is given by the same input voltage in an inverted form (*U*_{*op*2} = *- k·U*_{*in*1}).

The proposed circuit thereby comprises a differential connection between a single analog line 302 (or multiple digital control lines) at the voltage supply inputs of the front-end circuit 300' and an associated transducer element 110 (which may e.g. be given by a piezoelectric element 305) in the scanhead 100. Due to the differential circuit design, the proposed front-end circuit has no common ground potential. A front-end circuit according to the present invention may either be implemented using conventional transmit amplifiers 304a and 304b, in which a differential cable 307 is required between the front-end circuit 300' and the particular transducer elements 110 of the transducer array in the scanhead 100. On the other hand, coaxial cables are traditionally used, which are not differential. Therefore, some other type of cables is needed, such as e.g. twisted-pair cables.

Another preferred implementation would be to implement bridged transmit amplifiers 304a and 304b in the scanhead 100. In this case, said amplifiers may be connected to the transducer elements 110 via integrated flex circuits. In this case, it would be easy to design flex circuits that directly connect to the transducer elements 110.

As illustrated in Fig. 3b, another important aspect of the invention is to provide a direct electrical connection 307 between the output port of each transmit amplifier 304a, 304b and each associated transducer element 110. It should be noted that a transformer is usually placed in-between when electrical isolation or conversion from differential to single-ended is required. However, it has been realized that due to the proximity of the components in the scanhead 100 the length of the wires making such connections are sufficiently small to do the direct differential connection to the scanhead elements and remove the need for the transformers.

It should be noted that Fig. 3b only shows the proposed circuit design for a single channel and a single transducer element 110 which, for a circuit design with more than one channel and more than one transducer element in the scanhead 100, would have to be reproduced for all transducer elements in the transducer array. It should further be noted that, according to this invention, an ASIC in the scanhead is preferred which implements the functionality of front-end circuit 300' as depicted in Fig. 3b and may also incorporate a beamformer functionality (microbeamforming) which controls the ultrasound transducer probe 100 to generate focused or non-focused ultrasound beams and controls the beamforming of the receive channels. In the proposed design, the application-specific integrated circuit implementing front-end circuit 300' does not necessarily have to be in flip-chip configuration, at least not for regular scanhead arrays. Said ASIC (flip-chip configuration or not) may instead be mounted on a printed circuit board (PCB) assembly which may be connected to acoustic elements via flex circuits.

For ultrasound transducer probes where the front-end circuit is connected directly to the input ports of the transducer elements 110, a suitable acoustic design may be provided that makes it possible not to have a common ground.

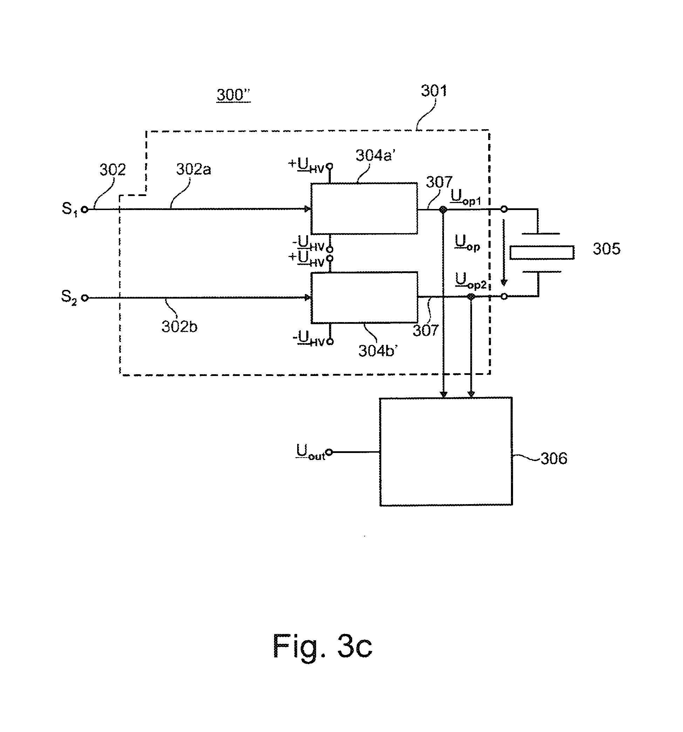

A digital implementation 300" of the ultrasound transducer probe's front-end circuit 300 as depicted in Fig. 3a is shown in Fig. 3c. This digital implementation comprises at least one transmit pulser 304a', 304b' integrated in each one of the two transmit branches 302a and 302b which are used for providing each transducer element 110 with the differential excitation or pulse voltage *Uₒₚ,* wherein the transmit pulser 304a' in a first one (302a) of these transmit branches provides a first output signal *U*_{*op*1} whose amplitude level is set by a first set of digital control signals *S*₁ fed to an input terminal of the transmit pulser 304a' in this first transmit branch 302a and wherein the transmit pulser 304b' in a second one (302b) of these transmit branches provides a second output signal *U*_{*op*2} whose amplitude level is set by a second set of digital control signals *S*₂ fed to an input terminal of the transmit pulser 304b' in this second transmit branch 302b.

Using a front-end circuit as described above for operating a medical probe, such as e.g. an ultrasound transducer probe (scanhead), effectively doubles the amplitude level of the front-end circuit's supply voltage and therefore quadruples the front-end circuit's achievable output power.

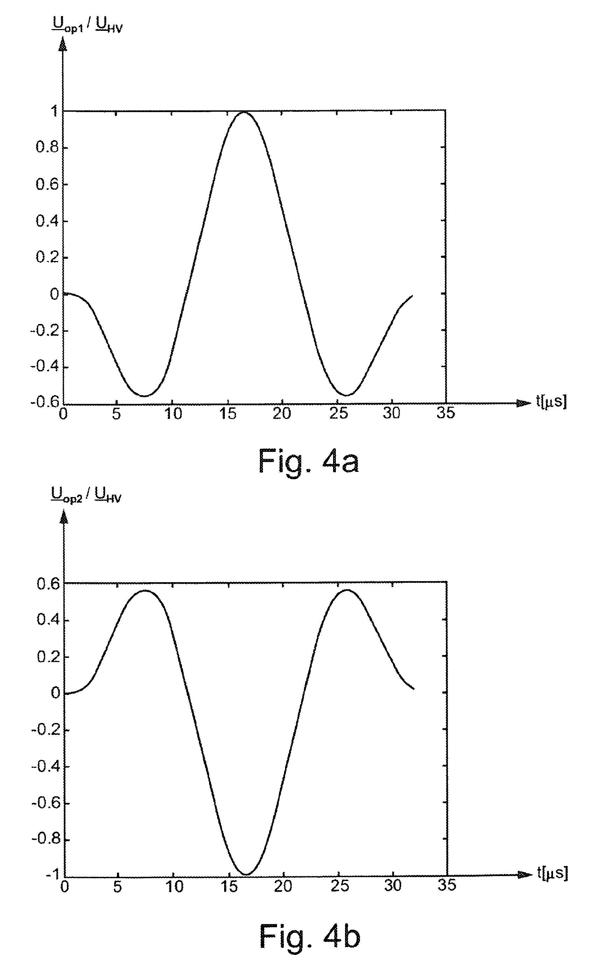

Fig. 4a shows the waveform of analog output voltage *U*_{*op*1} at the output terminal of the transmit amplifier 304a in the first transmit branch 302a of the ultrasound transducer probe's front-end circuit 300' according to the analog implementation as depicted in Fig. 3b. As can be taken from Fig. 4a, output voltage *U*_{*op*1} reaches the maximum positive voltage level (*U*_{*op*1,*max*} = +*U_{HV})* that can be handled by the IC fabrication process of an integrated circuit which realizes the functionality of the above-described front-end circuit.

Fig. 4b shows the waveform of analog output voltage *U*_{*op*2} at the output terminal of the transmit amplifier 304b in a second transmit branch 302b of the ultrasound transducer probe's front-end circuit 300' according to the analog implementation as depicted in Fig. 3b. It can be taken from Fig. 4b that output voltage *U*_{*op*2} reaches the minimum negative voltage level (*U*_{*op*2,*min*} = -*U_{HV})* that can be handled by the IC fabrication process.

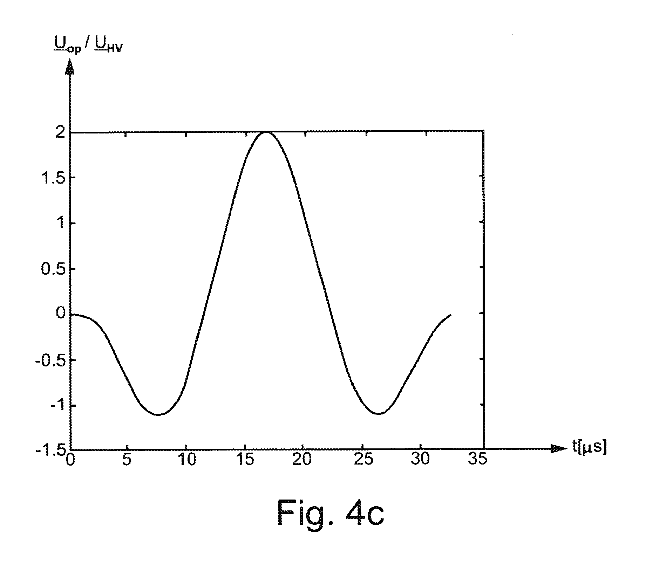

Fig. 4c shows the waveform of excitation or pulse voltage *Uₒₚ* for operating at least one differentially connected transducer element 305 of the ultrasound transducer probe's transducer array, wherein said excitation or pulse voltage is given by the difference of said first and second analog voltages *U*_{*op*1} and *U*_{*op*2}. As provided according to the present invention, excitation or pulse voltage *Uₒₚ* can thus be doubled without having to change the IC fabrication process.

### APPLICATIONS OF THE INVENTION

The invention can advantageously be applied in the field of compact ultrasound machines and other applications which require the use of a front-end integrated circuit that is fabricated using a relatively low voltage fabrication process or to reduce an ultrasound transducer probe's operating voltage for size, cost or safety reasons. The invention's main application is for low-cost, compact ultrasound machines where scanheads with an integrated beamforming functionality are used. For these applications, the present invention provides a means to use a relatively inexpensive acoustic design and serves for supplying a significantly higher excitation or pulse voltage given the voltage constraints of a given IC fabrication process.

While the present invention has been illustrated and described in detail in the drawings and in the foregoing description, such illustration and description are to be considered illustrative or exemplary and not restrictive, which means that the invention is not limited to the disclosed embodiments. Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. Furthermore, any reference signs contained in the claims should not be construed as limiting the scope of the invention.

### LIST OF REFERENCE SIGNS

- 100: Ultrasonic transducer assembly (also referred to as "ultrasound transducer", "ultrasound transducer probe" or "scanhead")
- 101: Face of ultrasound transducer probe 100
- 110: Array of piezoelectric elements (also referred to as "transducer elements"), which both transmit and receive ultrasonic waves
- 120: Single-ended cable
- 130: Base ultrasound system
- 132: Processing and control equipment
- 133: Display
- 200: Ultrasound transducer probe
- 210: Transducer array (comprising ultrasound transducer probe 200)
- 211: Transducer elements of transducer array 210
- 220: Single-ended cable
- 221-1: First transmit channel
- 221-n: n-th transmit channel
- 222a: Multiplexer
- 222b: Demultiplexer
- 232: Processing means
- 233: Analog-to-digital (A/D) converters
- 234: Digital delays
- 235: Summer
- 240-1: First sub-array of transducer array 210
- 240-n: *n*-th sub-array of transducer array 210
- 241: Pre-amplifier
- 242: Low-power analog delay
- 300: Front-end circuit (simplified embodiment)
- 300': Front-end circuit (analog implementation, more detailed embodiment)
- 300": Front-end circuit (digital implementation, more detailed embodiment)
- 301: Transmission stage, configured as a bridged or differential amplifier
- 302: Input voltage feeding line of front-end circuit 300'
- 302a: First transmit branch of front-end circuit 300, 300' or 300", used for supplying a first input voltage or voltage control signal *U*_{*in*1} or a first set of digital control signals S₁
- 302b: Second transmit branch of front-end circuit 300, 300' or 300", used for supplying a second input voltage or voltage control signal *U*_{*in*2} (wherein *U*_{*in*2} may be given by *U*_{*in*1} in an inverted form as supplied by inverter 303) or a second set of digital control signals S₂
- 303: Inverter or digital control circuit, integrated in second transmit branch 302b
- 304a: High-voltage transmit amplifier stage, integrated in the first transmit branch 302a of the analog front-end circuit 300'
- 304a': Transmit pulser stage, integrated in the first transmit branch 302a of the digital front-end circuit 300"
- 304b: High-voltage transmit amplifier stage, integrated in the second transmit branch 302b of the analog front-end circuit 300'
- 304b': Transmit pulser stage, integrated in the second transmit branch 302b of the digital front-end circuit 300"
- 305: Transducer element (e.g. a piezoelectric elements made of lead zirconate titanate or other material), belonging to an array of transducer elements which are integrated in scanhead 100
- 306: Differential reception stage
- 307: Differential cable, flex circuit or other electrical interconnect between differential amplifier stages 304a/b and transducer element 305
- *k*: Gain of amplifiers 304a and 304b
- *S₁*: First set of digital control signals, fed to high-voltage transmit amplifier stage 304a
- *S*₂: Second set of digital control signals, fed to high-voltage transmit amplifier stage 304b
- *t*: Continuous time variable
- *U*_{*in*1}: Analog input voltage or voltage control signal of front-end circuit 300 or 300'
- *U*_{*in*2}: Further analog input voltage or voltage control signal of front-end circuit 300 or 300'
- ±*U_{HV}*: Positive and negative supply voltage potential of high-voltage transmit amplifier stages 304a and 304b
- *Uₒₚ*: Excitation or pulse voltage of transducer element 305
- *U*_{*op*1}: Output signal of high-voltage transmit amplifier stage 304a
- *U*_{*op*1,*max*}: Maximum level of output signal *U*_{*op*1}
- *U*_{*op*2}: Output signal of high-voltage transmit amplifier stage 304b
- *U*_{*op*2,*min*}: Minimum level ofoutput signal *U*_{*op*2}
- *Uₒᵤₜ*: Output signal of receive amplifier 306 at the output port of front-end circuit 300, 300' or 300" (can either be single-ended or differential)

## Claims

1. An ultrasound transducer probe (100) comprising,
- an array of differentially connected transducer elements (110) for transmitting ultrasound transmit pulses and receiving echo signals in response to these transmit pulses,
- a front-end circuit (300, 300' or 300") comprising a transmission stage (301) with two separate transmit branches (302a, 302b) being respectively connected to a different terminal of each transducer element (110) for providing each of these transducer elements (110) with a differential excitation or pulse voltage *(Uₒₚ)* whose amplitude level is given by the difference (*U*_{*op*1}-*U*_{*op*1}) with *U*_{*op*1} and *U*_{*op*2} being given by the front-end circuit's input control signals *(U*_{*in*1}, *U*_{*in*2}) which are fed via the two transmit branches (302a, 302b) to the respective transducer element (110);
**characterized by**
- a bridged amplifier topology having at least one transmit pulser (304a', 304b') integrated in each one of the two transmit branches (302a, 302b) which are used for providing each transducer element (110) with the differential excitation or pulse voltage *(Uₒₚ),* wherein the transmit pulser (304a') in a first one (302a) of these transmit branches is adapted to provide a first output signal (*U*_{*op*1}) whose amplitude level is set by a first set of digital control signals (*S*₁) fed to an input terminal of the transmit pulser (304a') in this first transmit branch (302a) and wherein the transmit pulser (304b') in a second one (302b) of these transmit branches is adapted to provide a second output signal (*U*_{*op*2}) whose amplitude level is set by a second set of digital control signals (*S*₂) fed to an input terminal of the transmit pulser (304b') in this second transmit branch (302b).

2. An ultrasound transducer probe according to claim 1,
wherein the output ports of the transmit pulsers (304a', 304b') are connected by a flip chip, flex circuit or other type of interconnect to an associated transducer element (110) of said array.

3. An ultrasound transducer probe according to claim 2,
wherein the transmit pulsers (304a', 304b') are integrated in the ultrasound transducer probe (100).

4. An ultrasound transducer probe according to claim 3,
implemented as an application-specific integrated circuit of the ultrasound transducer probe (100).

5. An ultrasound transducer probe according to claim 4,
comprising a differential reception stage (306) which provides an output signal (*Uₒᵤₜ*) representing said echo signals.

6. An ultrasound transducer probe according to claim 5,
wherein said reception stage connects each terminal of the at least one transducer element (110) to an associated low-noise amplifier.

7. An ultrasound transducer probe according to claim 6,
wherein each transducer element (110) is realized as a piezoelectric element (305).

8. An ultrasound diagnostic imaging system, said system comprising an ultrasound transducer probe (100) as defined in any of claims 1 to 7.

9. An ultrasound diagnostic imaging system according to claim 8, equipped with an integrated microbeamformer system.

## Patentansprüche

1. Ultraschallwandlersonde (100), die Folgendes umfasst:
- eine Anordnung unterschiedlich verbundener Wandlerelemente (110) zum Übertragen von Ultraschallübertragungsimpulsen und zum Empfangen von Echosignalen in Reaktion auf diese Übertragungsimpulse,
- eine Eingangsschaltung (300, 300' oder 300"), die eine Übertragungsstufe (301) mit zwei einzelnen Übertragungszweigen (302a, 302b) aufweist, die mit je einem anderen Anschluss jedes Wandlerelementes (110) verbunden sind, damit jedes dieser Wandlerelemente (110) mit einer differentiellen Erregungs- oder Pulsspannung (Uₒₚ) versehen wird, deren Amplitudenpegel durch die Differenz (Uₒₚ₁-Uₒₚ₂) gegeben wird, wobei Uₒₚ₁ und Uₒₚ₂ durch die Eingangssteuersignale (Uᵢₙ₁, Uᵢₙ₂) der Eingangsschaltung gegeben werden, wobei diese Signale über die zwei Übertragungszweige (302a, 302b) dem betreffenden Wandlerelement (110) zugeführt werden;
**gekennzeichnet durch**
- die Topologie eines überbrückten Verstärkers mit wenigstens einem Übertragungspulsgeber (304a', 304b'), integriert in jede, der zwei Übertragungszweige (302a, 302b), die dazu verwendet werden, jedem Wandlerelement (110) mit der differentiellen Erregungs- oder Pulsspannung (Uₒₚ) zu versehen, wobei der Übertragungspulsgeber (304a') in einem ersten Zweig (302a) dieser Übertragungszweige dazu vorgesehen ist, ein erstes Ausgangssignal (Uₒₚ₁) zu liefern, dessen Amplitudenpegel durch einen ersten Satz digitaler Steuersignale (S₁) eingestellt wird, die einem Eingangsanschluss des Übertragungspulsgebers (304a') in diesem ersten Übertragungszweig (302a) zugeführt werden und wobei der Übertragungspulsgeber (304b') in einem zweiten Zweig dieser Übertragungszweige dazu vorgesehen ist, ein zweites Ausgangssignal (Uₒₚ₂) zu liefern, dessen Amplitudenpegel **durch** einen zweiten Satz digitaler Steuersignale (S₂) eingestellt wird, die einem Eingangsanschluss des Übertragungspulsgebers (304b') in diesem zweiten Übertragungszweig (302b) zugeführt werden.

2. Ultraschallwandlersonde nach Anspruch 1,
wobei die Ausgangsporte des Übertragungspulsgebers (304a', 304b') durch einen "Flip Chip", eine Flexschaltung oder einen anderen Verbindungstyp mit einem assoziierten Wandlerelement (110) der genannten Anordnung verbunden sind.

3. Ultraschallwandlersonde nach Anspruch 2,
wobei die Übertragungspulsgeber (304a', 304b') in der Ultraschallwandlersonde (100) integriert sind.

4. Ultraschallwandlersonde nach Anspruch 3,
implementiert als applikationsspezifische integrierte Schaltung der Ultraschallwandlersonde (100).

5. Ultraschallwandlersonde nach Anspruch 4,
die eine differenzielle Empfangsstufe (306) aufweist, die ein die genannten Echosignale darstellendes Ausgangssignal (Uₒᵤₜ) liefert.

6. Ultraschallwandlersonde nach Anspruch 5,
wobei die genannte Empfangsstufe jeden Anschluss des wenigstens einen Wandlerelementes (110) mit einem assoziierten rauscharmen Verstärker verbindet.

7. Ultraschallwandlersonde nach Anspruch 6,
wobei jedes Wandlerelement (110) als piezoelektrisches Element verwirklicht ist.

8. Diagnostisches Ultraschallbildformungssystem, wobei das genannte System eine Ultraschallwandlersonde (100) nach einem der Ansprüche 1 bis 7 aufweist.

9. Diagnostisches Ultraschallbildformungssystem nach Anspruch 8, ausgerüstet mit einem integrierten Mikrostrahlformungssystem.

## Revendications

1. Sonde à transducteur à ultrasons (100) comprenant :
- un réseau d'éléments de transducteur connectés de façon différentielle (110) pour transmettre des impulsions d'émission d'ultrasons et pour recevoir des signaux d'écho en réponse à ces impulsions d'émission,
- un circuit d'extrémité avant (300, 300'ou 300") comprenant un étage de transmission (301) avec deux branches de transmission séparées (302a, 302b) étant connectées respectivement à une borne différente de chaque élément de transducteur (110) pour munir chacun de ces éléments de transducteur (110) d'une tension d'excitation ou d'impulsion différentielle (*Uₒₚ*) dont le niveau d'amplitude est donné par la différence (*U*_{*op*1}-*U*_{*op*2}), *Uₒₚ₁* et *U*_{*op*2} étant données par les signaux de commande d'entrée du circuit d'extrémité avant *(U*_{*in*1}-*U*_{*in*2}) qui sont fournis par le biais des deux branches de transmission (302a, 302b) à l'élément de transducteur respectif (110) ;
**caractérisée par** :
- une topologie d'amplificateur ponté ayant au moins un générateur d'impulsions de transmission (304a', 304b') étant intégré dans chacune des deux branches de transmission (302a, 302b) qui sont utilisées pour munir chaque élément de transducteur (110) de la tension d'excitation ou d'impulsion différentielle (*Uₒₚ*), dans laquelle le générateur d'impulsions de transmission (304a') dans une première (302a) de ces branches de transmission est adapté de manière à fournir un premier signal de sortie (*Uₒₚ₁*) dont le niveau d'amplitude est réglé par un premier ensemble de signaux de commande numériques (*S₁*) qui sont fournis à une borne d'entrée du générateur d'impulsions de transmission (304a') dans cette première branche de transmission (302a) et dans laquelle le générateur d'impulsions de transmission (304b') dans une seconde (302b) de ces branches de transmission est adapté de manière à fournir un second signal de sortie (*U*_{*op*2}) dont le niveau d'amplitude est réglé par un second ensemble de signaux de commande numériques (S₂) qui sont fournis à une borne d'entrée du générateur d'impulsions de transmission (304b') dans cette seconde branche de transmission (302b).

2. Sonde à transducteur à ultrasons selon la revendication 1,
dans laquelle les orifices de sortie des générateurs d'impulsions de transmission (304a', 304b') sont connectés par une puce à protubérances, par un circuit flexible ou par un autre type d'interconnexion à un élément de transducteur associé (110) dudit réseau.

3. Sonde à transducteur à ultrasons selon la revendication 2,
dans laquelle les générateurs d'impulsions de transmission (304a', 304b') sont intégrés dans la sonde à transducteur à ultrasons (100).

4. Sonde à transducteur à ultrasons selon la revendication 3,
qui est mise en oeuvre en tant qu'un circuit intégré à application spécifique de la sonde à transducteur à ultrasons (100).

5. Sonde à transducteur à ultrasons selon la revendication 4,
comprenant un étage de réception différentiel (306) qui fournit un signal de sortie (*Uₒᵤₜ*) représentant lesdits signaux d'écho.

6. Sonde à transducteur à ultrasons selon la revendication 5,
dans laquelle ledit étage de réception connecte chaque borne de l'au moins un élément de transducteur (110) à un amplificateur à faible bruit associé.

7. Sonde à transducteur à ultrasons selon la revendication 6,
dans laquelle chaque élément de transducteur (110) est réalisé en tant qu'un élément piézoélectrique (305).

8. Système d'imagerie de diagnostic à ultrasons, ledit système comprenant une sonde à transducteur à ultrasons (100), telle qu'elle est définie dans l'une quelconque des revendications précédentes 1 à 7.

9. Système d'imagerie de diagnostic à ultrasons selon la revendication 8, qui est équipé d'un système intégré de formation de microfaisceau.
